# EUROPEAN PATENT SPECIFICATION

(11) **EP 2 294 067 B1**
(45) Date of publication and mention of the grant of the patent: **18.02.2015**
(21) Application number: 09738141.2
(22) Date of filing: 28.04.2009
(51) Int. Cl.: C07D 457/02, C07D 457/04, C07D 457/06, A61K 31/48, A61P 25/00

(54) **PREPARATION OF N-6 DEMETHYLATED, 9-10-DIHYDROLYSERGIC ACID ALKYL ESTERS**
HERSTELLUNG VON N-6-DEMETHYLIERTEN 9,10-DIHYDROLYSESÄUREALKYLESTERN
PREPARATION D'ESTERS ALKYLIQUES D'ACIDE 9-10-DIHYDROLYSERGIQUE DEMETHYLES EN N-6

(30) Priority: 29.04.2008 EP 08155371
(43) Date of publication of application: 16.03.2011
(73) Proprietor: LEK Pharmaceuticals d.d., 1526 Ljubljana (SI)
(72) Inventor: CASAR, Zdenko, 1526 Ljubljana (SI); MESAR, Tomaz, 1526 Ljubljana (SI)
(74) Representative: Prüfer & Partner GbR European Patent Attorneys
(86) International application number: PCT/EP2009/055117
(87) International publication number: WO 2009/133097

(56) References cited:
- EP-A1- 0 003 667
- A.M. CRIDER ET AL.: "Convenient synthesis of 6-nor-9,10-dihydrolysergic acid methyl ester" JOURNAL OF PHARMACEUTICAL SCIENCES, vol. 70, no. 12, 1981, pages 1319-1321, XP002495605 cited in the application
- MURUGAN RAMIAH ET AL: "Applications of dialkylaminopyridine (DMAP) catalysts in organic synthesis" ALDRICHIMICA ACTA, ALDRICH CHEMICAL CO., MILWAUKEE, US, vol. 36, no. 1, 1 January 2003 (2003-01-01), pages 21-27, XP009091740 ISSN: 0002-5100 cited in the application
- Satomi Imori ET AL: "Efficient Demethylation of N , N- Dimethylanilines with Phenyl Chloroformate in Ionic Liquids", Synlett, vol. 2006, no. 16, 1 September 2006 (2006-09-01), pages 2629-2632, XP055133773, ISSN: 0936-5214, DOI: 10.1055/s-2006-951490
- ASHFORD S W ET AL: "A Practical Synthesis of Cabergoline", THE JOURNAL OF ORGANIC CHEMISTRY, AMERICAN CHEMICAL SOCIETY, US, vol. 67, 1 January 2002 (2002-01-01), pages 7147-7150, XP002334751, ISSN: 0022-3263, DOI: 10.1021/JO0203847
- R.T. OWEN: "Pergolide Mesylate", DRUGS OF THE FUTURE, vol. 6, no. 4, 1981, pages 231-233,

## Description

### Field of the Invention

The present invention relates to a process for producing *N*-6 demethylated 9,10-dihydrolysergic acid alkyl esters comprising a step of *N*-6 demethylation of 9,10-dihydrolysergic acid alkyl esters with chloroformates, which may form a step in the synthesis of ergot alkaloid drugs.

### Background of the Invention

The *N*-6 demethylation of 9,10-dihydrolysergic acid alkyl esters (in the following sometimes abbreviated as DHLSAlkyl, for example its methylester (DHLSMe), may constitute a useful step in the chemical synthesis of various ergot alkaloid drugs which have a range of medical activities. A convenient synthesis for the *N*-6 demethylation was disclosed a few decades ago by A. M. Crider et al. in Journal of Pharmaceutical Sciences, Vol. 70 (12), pp. 1319-1321 (1981*).*

The object of the present invention is to provide an improved process for producing *N*-6 demethylated 9,10-dihydrolysergic acid alkyl esters, and to provide improved ergot alkaloid drugs and intermediate products therefor.

### Summary of the Invention

According to one aspect, the present invention provides a process for producing *N*-6 demethylated 9,10-dihydrolysergic acid alkyl ester, comprising a step of *N*-6 demethylation of a 9,10-dihydrolysergic acid alkyl ester with chloroformate, characterized that the *N*-6 demethylation step is performed in the presence of an organic base as an organic catalyst.

According to another aspect, the present invention provides a process for converting a compound of formula I (DHLSAlkyl) into a compound of formula II (DHLSAlkyl-F), 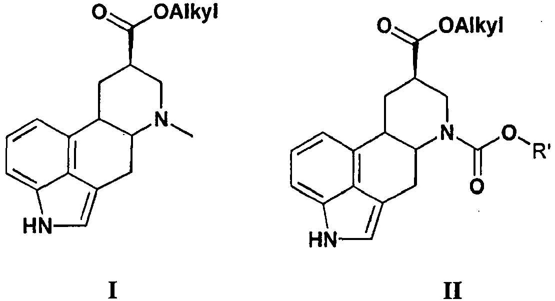 wherein R' is a substituted or unsubstituted hydrocarbon group, the process comprising the steps of:
▪ providing and preferably maintaining a reaction medium free of water and/or HCl, and
▪ reacting DHLSAlkyl with chloroformate being defined by the formula 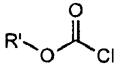 with R' having the aforementioned meaning in said reaction medium free of water and/or HCl, optionally in the presence of an organic base as an organic catalyst to obtain the compound of formula II (DHLSAlkyl-F), wherein at least one additional water and/or HCl removal step is performed.

In still another aspect, the present invention provides a process as defined above, wherein the ratio of chloroformate used in total compared to 9,10-dihydrolysergic acid alkyl ester used in total for the step of *N*-6 demethylation is less than 5 equivalents to 1 equivalent, respectively.

The process defined in the aspects above can be beneficially used in a process for the preparation of ergot alkaloid drugs.

The aforementioned compounds and drugs are obtained by providing corresponding reaction media respectively containing said compounds and drugs, e.g. the demethylation reaction medium from the *N*-6 demethylation of 9,10-dihydrolysergic acid alkyl ester of the invention, but being free, preferably entirely free of the afore-defined impurities. The desired compounds and drugs can then be isolated in pure form.

Hence, in a particular embodiment, the present invention provides a process for preparing an ergot alkaloid drug, comprising: a process as defined above for obtaining
an intermediate compound of formula II (DHLSAlkyl-F) or an intermediate compound of formula III (H-DHLSAlkyl), 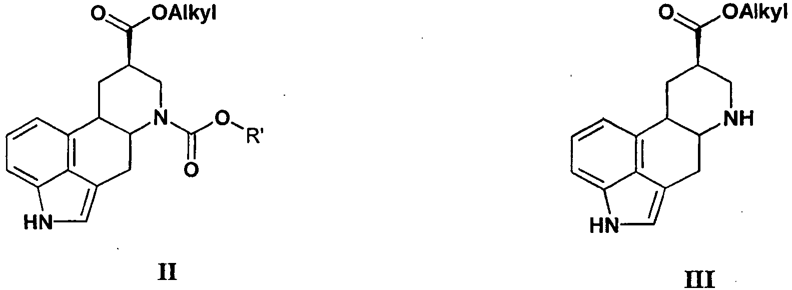 said intermediate compound of formula II (DHLSAlkyl-F) or of formula III (H-DHLSAlkyl) being free of any one of impurities of formula 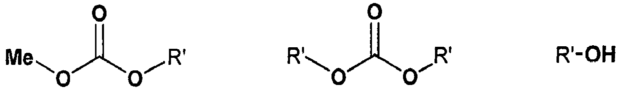 wherein R' is a substituted or unsubstituted hydrocarbon group, particularly wherein R' is defined by formula IV or V 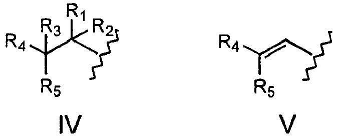 wherein R₁ and R₂, are the same or different and can be hydrogen atoms or are selected from halogens atoms (F, Cl, Br, I),
and wherein R₃, R₄, and R₅, are the same or different and can be hydrogen atoms or are selected from halogens atoms (F, Cl, Br, I), optionally substituted C₁ - C₈ alkyl, optionally substituted C₃-C₆ cycloalkyl, optionally substituted C₁ - C₈ alkenyl, and optionally substituted C₅-C₆ cycloalkenyl or aryl;
and carrying our further synthesis steps to obtain the ergot alkaloid drug.

The term "alkyl" used herein, if not specified otherwise, corresponds to the typical meaning of, for example, straight chain or branched chain, unsubstituted or substituted organic alkyl groups, possibly containing C=C double bonds and/or hetero atoms such as O, S, N with optional further substituents, suitably e.g. C1 to C6 alkyl, preferably methyl, ethyl, propyl, isopropyl, butyl, pentyl, hexyl, or the like.

The term "*N*-6 demethylation" used herein means replacement of methyl group by another group, more particularly it means replacement by a formate group being defined by 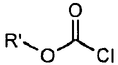 with R' having the aforementioned meaning, or a replacement by hydrogen.

Thus, the above-mentioned object is basically solved by the subject-matter of present claims 1, 5, 10, 12, 13 and 14. Preferred embodiments are set forth in sub-claims thereto.

### Brief Description of the Drawings

Fig. 1 shows process chemistry based on prior art, from which adaptations and optimizations according to an embodiment of the present invention become apparent;
Fig. 2 shows organocatalytic process chemistry replacing the inorganic bicarbonate system of Fig. 1, forming the basis for another embodiment of the present invention;
Fig. 3 illustratively shows a possible synthesis scheme for obtaining ergot alkaloid drugs, such as for example cabegoline or pergolide.
Fig. 4 shows a comparison of kinetics of DHLSMe conversion to DHLSMe-F for adapted and optimized standard (in sense of continous addition of TCECF), and for organocatalytic processes according to various embodiments of the present invention.

### Detailed Description of the Invention, its Advantages and Preferred Embodiments

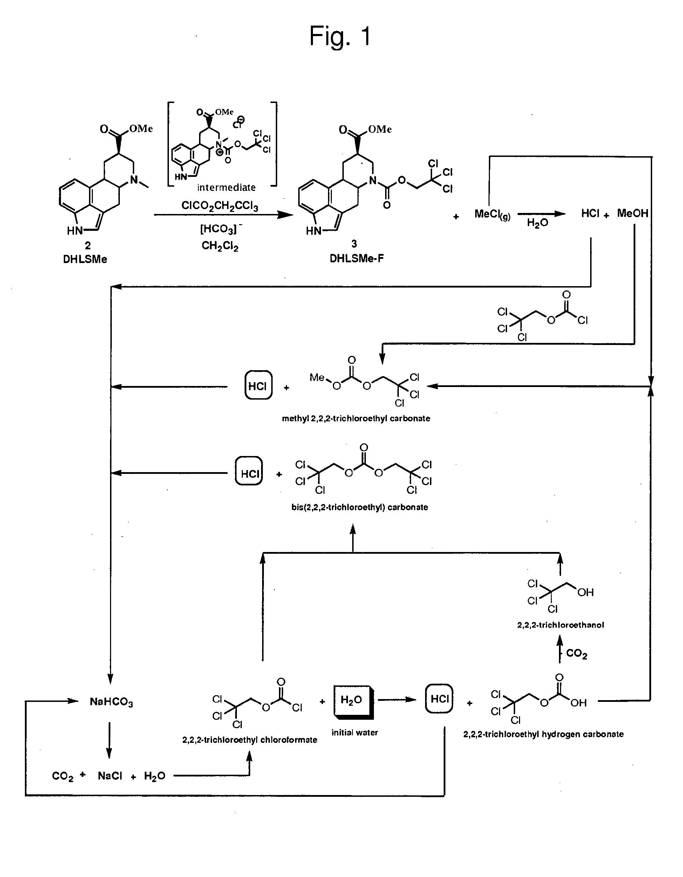

In the present invention, it was found that the synthesis system described by Crider et al. is not industrially applicable due to its unscalability from the laboratory scale to industrial scale. It was recognized that inorganic bicarbonates, as required in the synthesis route disclosed by Crider et al., exert a deleterious effect by entering a self-sustaining (driving, preserving) cycle of chemical reactions that produce and accumulate water, HCl and other side products such as organic carbonates in the system, which eventually blocks the demethylation reaction. Chemical reactions being presumably relevant in the reaction system of the demethylation step are shown in Fig. 1, as illustrated for the example where the chloroformate is embodied by trichloroethyl chloroformate (TCECF). Without proper adaptations and control of the reaction system, it occurs that per one cycle at least 3 molecules of water are released from an initial molecule of water (originating from the solvent or moisture) *via* release of 3 molecules HCl per cycle and its reaction with HCO₃⁻. Therefore, presence of water would be amplified and accumulated from initial water in the system during the reaction and would eventually block the demethylation reaction progress through released and co-accumulated HCl, the latter protonating the 6-*N* nitrogen atom of the basic ergoline structure and thus blocks it to react with the chloroformate (here TCECF). The water and HCl accumulation problems identified by the present inventors have found to become pronounced especially when the reaction system shall be scaled up.

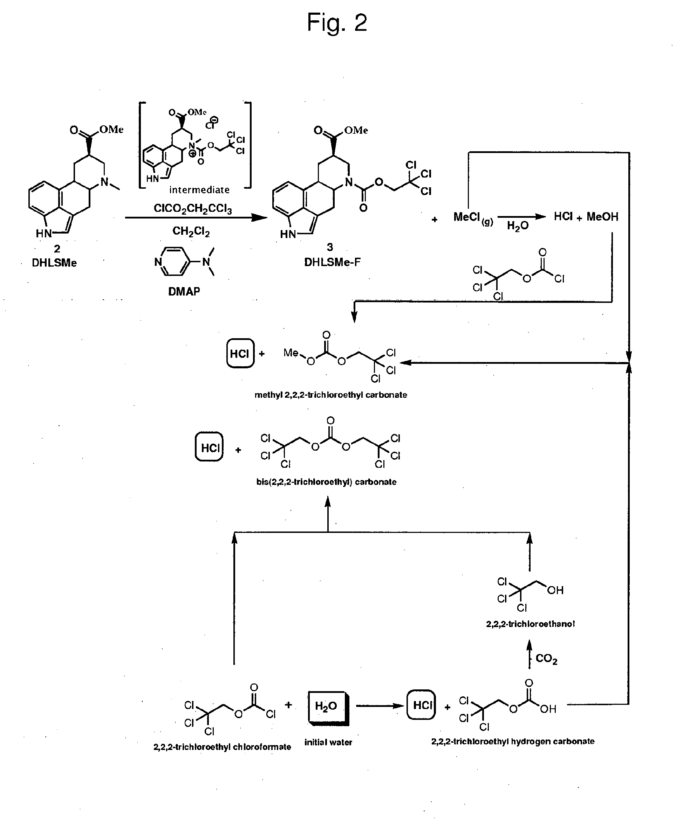

It was now surprisingly found, however, that when applying organic catalyst in the *N*-6 demethylation step, as shown in Fig. 2 by illustratively referring again to the use of trichloroethyl chloroformate (TCECF) reagent, a water release and thus water accumulation is prevented during its function. Consequently, HCl release and accumulation can be prevented or minimized as well. This leads to a significant increase of the yield of the product, especially on industrial scale, compared to the original procedure disclosed by Crider et al. Thus, according to one solution approach of the present invention, the *N*-6 demethylation step is performed in the presence of an organic catalyst. In this approach, potassium bicarbonate or other bicarbonates or possibly also other inorganic bases, and the problems associated therewith, can be surely avoided.

Moreover, the findings of the present invention provided another alternative solution approach of the problems associated with the synthesis system by Crider et al. Namely, if particular care is observed to ensure an absence and preferably an entire absence of initial water, the probability of HCl release and water accumulation reactions as shown in Fig. 1 is significantly reduced and minimized, even if an organic catalyst is not used. Therefore, providing a reaction medium free of water and/or HCl already from the beginning before the demethylation reaction is started to proceed by adding a relevant substrate, does have a remarkable and - especially when viewed from a laboratory scale - unpredictable effect of significantly increasing the yield of the product. This effect becomes substantial particularly on an industrial scale, when inorganic bases such as bicarbonates are used as released acid scavengers.

The solution approaches of the present invention described above can be advantageously combined.

When an organic catalyst is used for the *N*-6 demethylation step in the process according to the present invention, an organic compound having a proton acceptor function is selected. An organic compound having a proton acceptor function can act as the catalyst for the demethylation reaction. And it may capture any remaining HCl during the reaction. Accordingly, the organic catalyst typically is an organic base. Preferred organic catalyst according to the present invention is selected from the group consisting of sterically hindered organic amines, secondary and tertiary organic amines, aromatic amines and heterocyclic organic amines. Suitable examples of such organic amines include, but are not limited to triethylamine, pyridine and pyridine derivatives, substituted or unsubstituted forms of dialkylaminopyridine and particularly dimethylaminopyridine, *N*-ethylmorpholine, imidazole, *N*-ethyldiisopropylamine, *N*-ethylmorpholine triethylamine, 1,5-diazabicyclo[4.3.0]non-5-ene, and the like. The organic catalyst used according to the present invention is particularly selected form the group of compounds consisting of dimethylaminopyridine (DMAP) and DMAP analogues of the following general formulae: 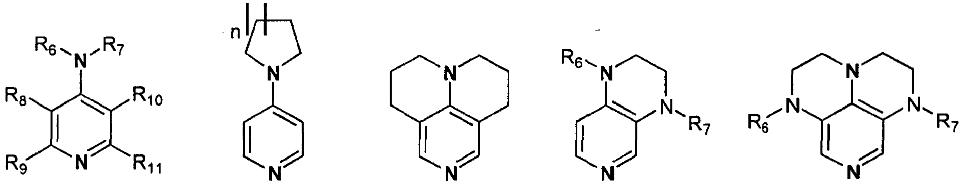 wherein R6 to R11 independently from each other denote H or C1 to C6 alkyl group, and n is 1, 2, 3, 4, 5 or 6.

Most preferably 4-(dimethylamino)pyridine (DMAP) 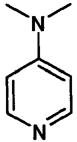 is used as the organic catalyst.

The use of DMAP and other analogs thereof as catalyst in organic synthesis such acylation reactions are described, for example, in D.J. Berry et al. ARKIVOC 2001 (i), pp. 201-226, and R. Murugan and E.F.V Scriven, Aldrichimica Acta 2003, 36, pp. 21 - 27.

When, in the alternative embodiment of the present invention, an organic catalyst is not used for the *N*-6 demethylation step or if it is only optional, it is important that a reaction medium free of water and/or HCl, more preferably entirely free of water is provided in advance of starting, or at the latest simultaneously with performing the actual demethylation step and specifically when converting a compound of formula I (DHLSAlkyl) into a compound of formula II (DHLSAlkyl-F). The importance of this condition is more pronounced, as the reaction system is scaled up from laboratory scale to bench scale and especially to industrial scale. This particularly applies when using a large scale at an amount of 9,10-dihydrolysergic acid alkyl ester (compound of formula I; DHLSAlkyl) above 1 kg, because scaling up increases the risk that any minute amounts of initial water is present in the system, which eventually accumulates further water molecules and releases further molecules of HCl per reaction cycle when bases like alkali metal bicarbonates, other than organic catalysts, are used. Thus, it has been found that if water present in the reaction system would be removed completely, release of HCl can be effectively prevented and thus problems of scaling up of the reaction system can be avoided even without using organic catalysts for the *N*-6 demethylation step. As particularly effective means to provide a reaction medium free of water and/or HCl, the medium can be subjected to purging with nitrogen or any other inert gas, and/or subjected to evacuation. This measure can be preferably carried out in advance of adding DHLSAlkyl into the reaction system,

Preferably, it is observed that a reaction medium free of water and/or HCl is not only provided in the starting materials or simultaneous with adding co-reactants, but also is maintained throughout the *N*-6 demethlyation reaction. For this purpose, at least one water and/or HCl removal step is provided, such as degasing by nitrogen flow or purge or by vacuum evacuation, which may be repeatedly performed. At least one water and/or HCl removal step is preferably performed after chloroformate has been added to a water-free solvent, by which measure a majority or all of possibly generated HCl is removed from the reaction system. Any remaining traces of HCl may be further captured by the base catalyst. Another suitable point of time for repeating a water and/or HCl removal step is when DHLSAlkyl is added and/or subsequent of its addition. Thus, according to preferred embodiment, the process of converting a compound of formula I into a compound of formula II comprises the steps of:
- providing a reaction medium free of water and/or HCl,
- before DHLSAlkyl is added, chloroformate is added to the reaction medium, and the reaction medium is refluxed,
- after chloroformate addition and medium reflux, the reaction medium is optionally but preferably purged with nitrogen, or is subjected to evacuation,
- DHLSAlkyl is then added to the obtained mixture, and
- the obtained mixture is subjected to reaction for time sufficient to give DHLSAlkyl-F (*N*-6 demethylated) product.

A preferred chloroformate reactant is defined by 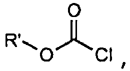 wherein R' is a substituted or unsubstituted hydrocarbon group, to give the corresponding demethylated DHLSAlkyl-F. Preferred R' residues are "alkyl", more preferably halogenated alkyl or vinyl. Particularly R' is defined by formula IV or V 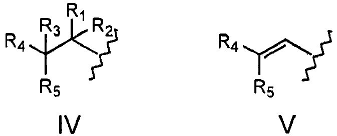 wherein R₁ and R₂, are the same or different and can be hydrogen atoms or are selected from halogens atoms (F, Cl, Br, I),
and wherein R₃, R₄, and R₅, are the same or different and can be hydrogen atoms or are selected from halogens atoms (F, Cl, Br, I), optionally substituted C₁ - C₈ alkyl, optionally substituted C₃-C₆ cycloalkyl, optionally substituted C₁ - C₈ alkenyl, and optionally substituted C₅-C₆ cycloalkenyl or aryl.

According to a particularly preferred embodiment of the present invention, both aspects of the invention are combined to provide an optimized reaction system which is most suitable for an up scaled *N*-6 demethylation of 9,10-dihydrolysergic acid alkyl esters. That is, a reaction medium free of water and/or HCl is provided, and the reaction medium is supplemented with an organic catalyst as described above at a desired point of time. A reaction medium containing chloroformate is preferably subjected to heat treatment, preferably under reflux, for some periods of time, for example at least 1, preferably at least 2 and more preferably at least 3 hours. Optionally, after cooling, the reaction medium can be purged with inert gas flow such as nitrogen purging and/or can be subjected to evacuation. Subsequently, organic catalyst as described above may be added at this point and may be mixed with the reaction medium for a period of time, optionally followed by another water and/or HCl removal step such as inert gas purging and/or evacuation. In accordance with the preferred embodiment, not before then the substrate DHLSAlkyl is added to start with the actual demethylation reaction.

In another optimization of a demthylation step according to a preferred embodiment of the present invention, which can be used with either embodiment described above, or both, chloroformate is continuously added to the reaction system. The term "continuously added" means that either a given amount of chloroformate is slowly and gradually added, or a given amount of chloroformate is added intermettantly but in multiple portions such as in two, preferably in three and more preferably in four or more portions.

Subsequent to the *N-*6 demethylation reaction, the carbamate group of the compound of formula II (DHLSAlkyl-F) can be subjected to further reactions in accordance with the options and choice of the person skilled in the art, such as the intended further synthesis route to obtain a desired ergot alkaloid drug. The carbamate group of the compound of formula II (DHLSAlkyl-F) may be cleaved by methods known in the art, to give the compound of formula III (H-DHLSAlkyl). The compound of formula III (H-DHLSAlkyl) can likewise be subjected to further reaction and synthesis steps to obtain various ergot alkaloid drugs, by methods known or apparent to the person skilled in the art.

A particularly advantageous embodiment of the present invention resides in that the molar ratio of co-reactant chloroformate used in total compared to 9,10-dihydrolysergic acid alkyl ester used in total for the step of *N*-6 demethylation can be significantly reduced in comparison with the prior art. Surprisingly, the process of present invention enables a reduction of the aforementioned ratio to less than 5 equivalents chloroformate to 1 equivalent of 9,10-dihydrolysergic acid alkyl ester in the *N-*6 demethylation step.

Hence, the present invention provides a robust, reliable, economic and scalable process for *N*-6 demethylation of 9,10- dihydrolysergic acid alkyl esters with chloroformate, with particular significance of increased yield of the product on an industrial scale. Thus, the process according to the present invention is particularly suitable to be performed at a large scale by using an amount of 9,10- dihydrolysergic acid alkyl ester above 1 kg, more preferably above 2 kg and even above 4 kg. Following the concept of the present invention enables the demethylation step for obtaining an intermediate compound of formula II (DHLSAlkyl-F) to be terminated in less than 24 hours.

Therefore, further particularly advantageous aspect of the present invention is the use of the process according to the present invention as described above in a process for the preparation of ergot alkaloid drugs. The advantages of the present invention are variable for the whole preparation scheme of total ergot alkaloid drug synthesis.

A further advantage and particularly preferred embodiment of the present invention is that the intermediate compounds of DHLSAlkyl-F or H-DHLSAlkyl, and accordingly any final ergot alkaloid drug obtained therefrom, are feasible which are free, beneficially entirely free of impurities of the following formulae, which otherwise would appear by following conventional synthesis routes: 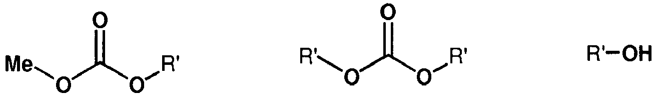 wherein R' is the hydrocarbon group derived from the chloroformate reactant. Specifically, R' is as defined above.

Based on the preferred embodiment of the present invention to use 2,2,2-trichloroethyl chloroformate (TCECF) as chloroformate for the *N*-6 demethylation of 9,10-dihydrolysergic acid alkyl esters, the intermediate product defined by either formula II or III (DHLSAlkyl-F or H-DHLSAlkyl), and the ergot alkaloid drugs are free and preferably entirely free of the following corresponding chlorinated impurities:

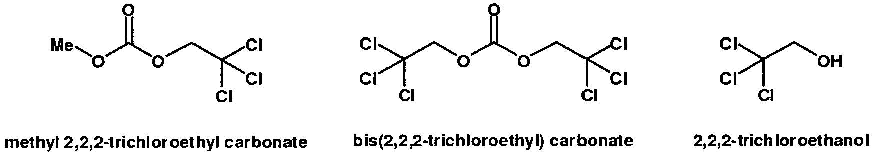

As used herein, "entirely free" means non-detectable by methods like GC, HPLC or mass spectrometry. As a suitable reference method for detectability, GC can be used.

An advantage of the present invention resides in that when the indicated intermediate compounds of DHLSAlkyl-F or H-DHLSAlkyl obtained by a process mentioned above are provided in a status free, preferably entirely free of the above defined impurities, it is possible to more surely obtain a correspondingly pure end product, especially the desired ergot alkaloid drug. Avoiding the formation of the mentoined impurities particularly contributes to not affecting further reaction steps, and avoiding generation of further impurities.

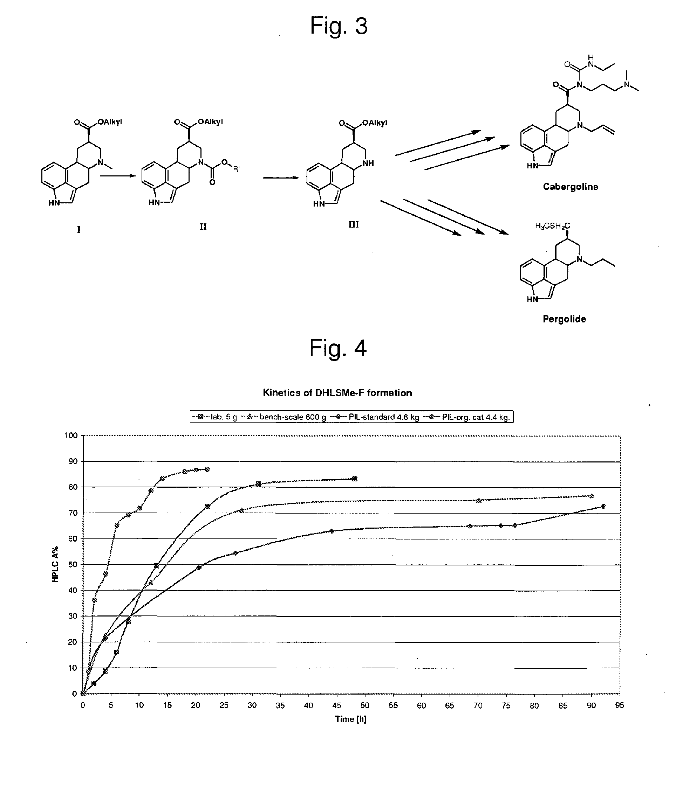

A possible synthesis scheme for obtaining ergot alkaloid drugs, such as preferably cabegoline or pergolide, are illustratively shown in Fig. 3, wherein the synthesis routes proceed by using compounds of formulae I, II, and III. According to the preferred embodiment, alkyl is methyl (Me), and thus the synthesis of ergot alkaloid drugs proceeds via DHLSMe-F and H-DHLSMe.

The present invention will be described in more detail in the following by reference to illustrative examples, which however are given for exemplary purposes only and shall not be interpreted in any limiting manner. Rather, the skilled person may use modifications or find equivalents within ordinary skill. For example, the use of the preferred chloroformate 2,2,2-trichloroethyl chloroformate (TCECF) is described in the examples and the Figures, while the skilled person will appreciate that alternative chloroformates as generically described above can be correspondingly used as well.

### Reference Examples

### Repeated literature procedure from A. M. Crider et al., J. Pharm. Sci. 1981, 70, 1319-1321.

### 6-Nor-6-(2,2,2-trichloroethoxycarbonyl)-9,10-dihydrolysergic Acid Methyl Ester

A mixture of 9,10-dihydrolysergic acid methyl ester (0.425g, 0.0015 mole), potassium bicarbonate (0.729 g, 0.0073 mole), and 2,2,2,trichloroethyl chloroformate (0.619 g, 0.0029 mole) in 60 mL of absolute methylene chloride was refluxed for 24 hours. 2,2,2-Trichloroethyl chloroformate (1.48 g, 0.0070 mole) was added and refluxing was continued for an additional 24 hours. The reaction mixture was cooled, filtered, and evaporated under reduced pressure to yield a white solid. Recrystalization from absolute methanol gave 0.602 g (90 %) of crystaline product, mp 208-210°; IR(KBr): 3485 (N-H, indole) and 1740 (C=O, ester an carbamate) cm⁻¹; NMR (dimethyl sulfoxide-d₆): δ 3.70 (s, 3H, COOCH₃), 4.90 (s, 2H; OCH₂CCl₃), 6.67-7.20 (m, 4H, aromatic), and 7.27 (s, 1 H, NH). Anal.-Calc. For C₁₉H₁₉Cl₃N₂O₄: C, 51.19; H, 4.30; Cl, 23.86; N, 6.29. Found: C, 51.14; H, 4.20; Cl, 24.21; N, 6.13.

Two batches were performed on 2.13 g scale:
2.13 g DHLSMe
3.645 g KHCO₃
300 mL DCM
2.01 mL TCECF reflux 24 h
4.80 mL TCECF reflux 24 h

### First batch

**Kinetics of the reaction:**

| **t (h)** | **DHLSMe** [HPLC A%] | **DHLSMeF** [HPLC A%] |
|---|---|---|
| 4 | 93.69 | 5.55 |
| 24 | 81.70 | 15.54 |
| 29 | 73.71 | 25.32 |
| 48 | 62.95 | 35.85 |

### Second batch

**Kinetics of the reaction:**

| **t (h)** | **DHLSMe** [HPLC A%] | **DHLSMeF** [HPLC A%] |
|---|---|---|
| 4 | 93.08 | 5.53 |
| 24 | 83.77 | 15.53 |
| 29 | 73.53 | 25.54 |
| 48 | 49.38 | 50.62 |

The reaction mixture was cooled, filtered, and evaporated under reduced pressure to yield a white solid.
Recrystalization from absolute methanol gave:
First batch: 1.02 g (30 %) HPLC: 99.67 area%
Second batch: 1.16 g (35 %) HPLC: 99.40 area%
of crystalline product.

### Example 1

### Optimized chemical reaction system, lab scale (5.0 g)

5.0 g (17.58 mmole) of 9,10-dihydrolysergic acid methyl ester was dissolved in 200 mL of absolute methylene chloride, after 7.2 g (85.8 mmol) of sodium bicarbonate were added and mixture was heated to reflux. Then 16.5 mL (25.39 g, 120 mmol) of 2,2,2,trichloroethyl chloroformate were slowly added in 24 hours. After complete addition the reaction mixture was refluxed further for 24 hours. After 48 hours the reaction mixture was cooled, filtered over aluminium oxide (10 g), washed with 150 mL of methylene chloride and evaporated under reduced pressure and 50 mL of absolute methanol were added and stirred for 2 hours. Crystalline product was filtered and dried to yield a 5.92 g (76 %) of product as a white solid.

**Kinetics of the reaction:**

| t [h] | DHLSMe [HPLC A%] | DHLSMe-F [HPLC A%] |
|---|---|---|
| 2 | 95.35 | 3.95 |
| 4 | 90.45 | 8.68 |
| 6 | 84.00 | 16.00 |
| 8 | 71.59 | 27.81 |
| 13 | 49.95 | 49.41 |
| 22 | 23.98 | 72.58 |
| 31 | 17.27 | 81.09 |
| 48 | 15.69 | 83.25 |

### Example 2

### Optimized chemical reaction system, bench-scale (600 g):

600 g (2.11 mole) of 9,10-dihydrolysergic acid methyl ester was dissolved in 24 L of absolute methylene chloride, after 864 g (10.3 mmol) of sodium bicarbonate were added. The obtained mixture was heated to reflux and ca. 2 L of methylene chloride was distilled off. Then 2.515 L (3.87 kg, 18.3 mol) of 2,2,2,trichloroethyl chloroformate were added in four portions (t₀ₕ = 640 mL, t_{11.5h} = 625 mL, t₂₈ₕ = 625 mL, t₇₀ₕ = 625 mL). Reaction mixture was refluxed for 90 hours, then the reaction mixture was cooled, filtered over aluminium oxide (6.2 kg), washed with 20 L of methylene chloride and evaporated under reduced pressure to 4 L. After 4.5 L of n-hexane were added and stirred for 2 hours at 0 °C. Crystalline product was filtered and dried to yield a 553 g (59 %) of white solid.

**Kinetics of the reaction:**

| t [h] | DHLSMe [HPLC A%] | DHLSMe-F [HPLC A%] |
|---|---|---|
| 4 | 75.15 | 22.47 |
| 12 | 54.02 | 42.98 |
| 28 | 26.53 | 71.05 |
| 70 | 21.45 | 75.04 |
| 72.5 | 19.45 | 71.35 |
| 90 | 17.90 | 76.80 |

### Example 3

### Optimized chemical reaction system, industrial scale (4.6 kg):

A dry nitrogen inertized 250 L reactor was charged with 4.6 kg DHLSMe, 6.6 kg sodium bicarbonate and 210 kg of dry dichloromethane. Then 2,2,2-trichloroethyl chloroformate (TCECF) (7.0 kg) was charged into the reactor. The mixture was heated to reflux and stirred at reflux for 92 hours. Additional quantity (18.5 kg) of 2,2,2-trichloroethyl chloroformate (TCECF) was charged into the reactor during the stirring at reflux. After the reaction mixture was cooled to 10 ± 5 °C and filtered through a pad of alumina. Fractions containing a product were collected, concentrated and cooled 10 ± 5 °C. Then heptane is added to the concentrated solution and the precipitated product was collected by centrifugation. After drying 4.1 kg (56 %) of DHLSMe-F is obtained.

**Kinetics of the reaction:**

| t [h] | DHLSMe [HPLC A%] | DHLSMe-F [HPLC A%] |
|---|---|---|
| 0 | 100 | 0 |
| 4 | 78,16 | 21,27 |
| 20,5 | 50,98 | 48,74 |
| 27 | 45,35 | 54,34 |
| 44 | 36,73 | 63,01 |
| 68,5 | 34,73 | 64,98 |
| 74 | 34,53 | 65,18 |
| 76,5 | 34,2 | 65,43 |
| 92 | 26,84 | 72,7 |

### Example 4

### Organocatalytic chemical reaction system, industrial scale (4.4 kg):

The following reaction scheme shows the principle applied in Example 4 of *N*-6 demethylation of 9,10-dihydrolysergic acid alkyl esters with chloroformates in the presence of DMAP.

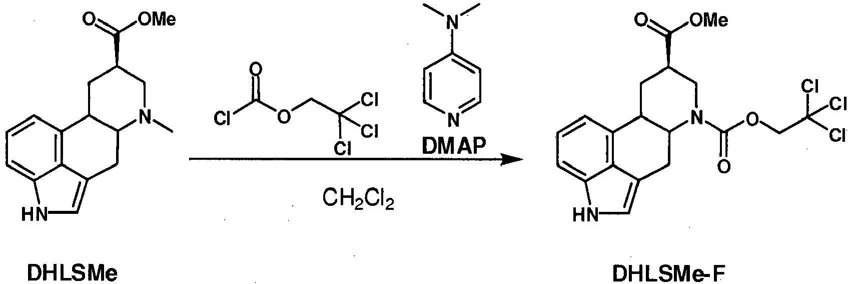

In the present example, 2,2,2-trichloroethyl chloroformate was used (i.e. R' is 2,2,2-trichloroethyl).

A 250 L reactor containing a 186 kg of dry dichlorometane was charged with 15.4 kg 2,2,2-trichloroethyl chloroformate (TCECF) at 20 ± 5 °C. The mixture was heated to reflux and refluxed for at least 3 hours. After the mixture was cooled to 20 ± 5 °C. The ractor was purged with nitrogen for at least 15 min. and charged with 88 g of DMAP at 20 ± 5 °C. The obtained mixture was then stirred at 20 ± 5 °C for at least 1 hour. The ractor was purged with nitrogen for at least 15 min. and charged with 4.4 kg of DHLSMe at 20 ± 5 °C. The obtained mixture was then heated to reflux and refluxed for 22 hours. After the reaction mixture was cooled to 10 ± 5 °C and filtered through a pad of alumina. Fractions containing a product were collected, concentrated and cooled 10 ± 5 °C. Then heptane is added to the concentrated solution and the precipitated product was collected by centrifugation. After drying 5.25 kg (76 %) of DHLSMe-F is obtained. DHLSMe-F assay is 100 % determined by HPLC.

**Kinetics of the reaction:**

| t [h] | DHLSMe [HPLC A%] | DHLSMe-F [HPLC A%] |
|---|---|---|
| 0 | 100 | 0 |
| 1 | 90,87 | 8,71 |
| 2 | 63,55 | 36,10 |
| 4 | 53,1 | 46,32 |
| 6 | 34,28 | 65,07 |
| 8 | 30,2 | 69,14 |
| 10 | 27,12 | 71,77 |
| 12 | 20,38 | 78,44 |
| 14 | 15,98 | 83,34 |
| 18 | 12,39 | 86,01 |
| 20 | 12,08 | 86,62 |
| 22 | 11,67 | 86,90 |

### Example 5

Molar ratios of co-reactants, kinetics any yields of the chemical reaction systems used in the Reference Example and in the Examples 1 to 4 were followed and monitored.

Advantages of organocatalytic process over standard process are presented in Table 1 which presents comparison of yields, reaction time and reagent stoichiometry (molar ratio between TCECF and DHLSMe).

**Table 1**

| | **Type and scale of reaction** | **Reaction time [h]** | **Yield [%]** | **Molar ratio TCECF/DHLSMe** |
|---|---|---|---|---|
| Reference Ex. | Literature data, lab scale (0.425 g) | 48 | 90 | 6.6 : 1 |
| Reference Ex. | Repeated literature data, lab scale (2.13 g) | 48 | 30-35 | 6.6 : 1 |
| Example 1 | Optimized, lab scale (5.0 g) | 48 | 76 | 6.8 : 1 |
| Example 2 | Optimized, bench-scale (600 g) | 90 | 59 | 8.7 : 1 |
| Example 3 | Optimized, industrial scale (4.6 kg) | 92 | 57 | 7.4 : 1 |
| Example 4 | Organocatalysis, industrial scale (4.4 kg) | **22** | **76** | **4.7 : 1** |

Further, comparison of kinetics for conversion of DHLSMe to DHLSMe-F is presented in Figure 4 (scale is based on the quantity of starting 9,10-dihydrolysergic acid methyl ester = DHLSMe entering the reaction) for adapted and optimized versions of standard reaction using inorganic bicarbonate base on lab-scale (5 g), bench-scale (600 g), industrial-scale = PIL-standard (4.6 kg) and by applying organocatalysis with DMAP on industrial scale = PIL-org. cat. (4.4 kg).

From Figure 4 significantly improved effects of the process according to the present invention, especially when using organocatalysis over standard process are clearly visible. Example 4 provides highest conversion of DHLSMe to DHLSMe-F in shortest time.

### Example 6

### Comparison Prior-art process and application of organic base catalyst

The following both experiments were performed on the same scale, the same solvent and temperature regime were applied, all other reagents were added in the same amount and the reaction time was the same.

| | KHCO₃ | | | | DMAP | | | |
|---|---|---|---|---|---|---|---|---|
| Procedure | A 2000 ml flask containing a 1400 mL of dry dichlorometane 10 g of DHLSMe (toluene solvate), 17.15 g of KHCO₃ and 9.25 mL of 2,2,2-trichloroethyl chloroformate (TCECF) were added. The mixture was heated to reflux and refluxed for 24 hours. 22 mL of 2,2,2-trichloroethyl chloroformate (TCECF) was added and refluxing was continued for additional 24 hours. After the reaction mixture was cooled, filtered and evaporated under reduced pressure to yield a white solid. Product was crystallized from 120 mL of methanol. | | | | A 2000 ml flask containing a 1400 mL of dry dichlorometane 10 g of DHLSMe (toluene solvate), 200 mg of DMAP and 9.25 mL of 2,2,2-trichloroethyl chloroformate (TCECF) were added. The mixture was heated to reflux and refluxed for 24 hours. 22 mL of 2,2,2-trichloroethyl chloroformate (TCECF) was added and refluxing was continued for additional 24 hours. After the reaction mixture was cooled, filtered and evaporated under reduced pressure to yield a white solid. Product was crystallized from 120 mL of methanol. | | | |
| Reactants | DCM 1400 mL | | | | DCM 1400 mL | | | |
| | DHLSMe 10 g (87.4 %); | | 30.3 mmol; | 1.00 eq | DHLSMe 10 g (87.4 %); | | 30.3 mmol; | 1.00 eq |
| | TCECF (9.25 + 22 mL); | | 232 mmol; | 7.73 eq | TCECF (9.25 + 22 mL); | | 232 mmol; | 7.73 eq |
| | KHCO₃ 17.15 g; | | 171 mmol; | 5.64 eq | DMAP 200 mg; | | 1.64 mmol; | 0.05 eq |
| m | 8.01 g | | | | 9.52 g | | | |
| HPLC | DHLSMe-F | - 98.84 area% | | | DHLSMe-F | - 96.50 area% | | |
| | DHLSMe | - 1.16 area% | | | DHLSMe | - 3.50 area% | | |
| Assay (HPLC) | 83.93 % (low assay is due to the carbonate impurities which are generated in prior-art process) | | | | 101.25 % | | | |
| Yield (mol.) | = m × Assay = 49.1 % | | | | = m × Assay = 70.4 % | | | |

The above direct comparison clearly demonstrates favorable effect of organic base catalyst on the outcome of the reaction. In particular, application of organic base catalyst instead of KHCO₃ in the process described in A. M. Crider et al. in Journal of Pharmaceutical Sciences, Vol. 70 (12), pp. 1319-1321 (1981) has significant advantage. This advantage is reflected in 70.4 % yield obtained when e.g. DMAP catalyst is used compared to 49.1 % yield by prior-art process where KHCO₃ is used. In addition, the prior-art product has low assay due to the high level of carbonate impurities which are not detectable by HPLC.

### Example 7

### Process according to the present invention with effect of water elimination

The use of organic catalyst can be further improved by appropriate design of the overall process by total elimination of water as disclosed in the process described below. Both experiments were performed on the same scale, the same solvent and temperature regime were applied, all other reagents were added in the same amount and the reaction time was the same. This clearly demonstrates favorable effect of DMAP catalyst on the outcome of the reaction.

| | KHCO₃ | | | | DMAP | | | |
|---|---|---|---|---|---|---|---|---|
| Procedure | A 1000 mL flask containing a 560 mL of dry dichlorometane was charged with 22.2 mL (35 g) 2,2,2-trichloroethyl chloroformate (TCECF) at 20 °C. The mixture was heated to reflux and refluxed for 1 hour. After the mixture was cooled to 20 °C. The flask was purged with nitrogen and charged with 17.15 g of KHCO₃ at 20 °C. The obtained mixture was then stirred at 20 °C for 15 min. The flask was purged with nitrogen for 15 min. and charged with 10 g of DHLSMe (toluene solvate) at 20 °C. The obtained mixture was then heated to reflux and refluxed for 22 hours. After the reaction mixture was cooled to 10 ± 5 °C and filtered through a pad of alumina 1-2 cm. Dichloromethane was evaporated off and 200 mL of *n*-heptane was added and the precipitated product was collected by filtration. After drying 13.70 g of DHLSMe-F is obtained. | | | | A 1000 mL flask containing a 560 mL of dry dichlorometane was charged with 22.2 mL (35 g) 2,2,2-trichloroethyl chloroformate (TCECF) at 20 °C. The mixture was heated to reflux and refluxed for 1 hour. After the mixture was cooled to 20 °C. The flask was purged with nitrogen and charged with 200 mg of DMAP at 20 °C. The obtained mixture was then stirred at 20 °C for 15 min.. The flask was purged with nitrogen for 15 min. and charged with 10 g of DHLSMe (toluene solvate) at 20 °C. The obtained mixture was then heated to reflux and refluxed for 22 hours. After the reaction mixture was cooled to 10 ± 5 °C and filtered through a pad of alumina 1-2 cm. Dichloromethane was evaporated off and 200 mL of *n*-heptane was added and the precipitated product was collected by filtration. After drying 13.55 g of DHLSMe-F is obtained. | | | |
| Reactants | DCM 560 mL | | | | DCM 560 mL | | | |
| | DHLSMe 10 g (87.4 %); | | 30.3 mmol; | 1.00 eq | DHLSMe 10 g (87.4 %); | | 30.3 mmol; | 1.00 eq |
| | TCECF 22.2 mL; | | 164.9 mmol; | 5.44 eq | TCECF 22.2 mL; | | 164.9 mmol; | 5.44 eq |
| | KHCO₃ 17.15 g; | | 171 mmol; | 5.64 eq | DMAP 200 mg; | | 1.64 mmol; | 0.05 eq |
| m | 13.70 g | | | | 13.55 g | | | |
| HPLC | DHLSMe-F | - 69.71 area% | | | DHLSMe-F | - 98.32 area% | | |
| | DHLSMe | - 30.21 area% | | | DHLSMe | - 1.68 area% | | |
| Assay | 73.77 % (low assay is due to the unreacted DHLSMe and carbonate impurities which are generated in prior-art process) | | | | 97.40 % | | | |
| Yield (mol.) | = m × Assay = 73.8 % | | | | = m × Assay = 96.3 % | | | |

When the new process with water elimination was used, even when conventional base KHCO₃ was applied, yield was as high as 73.8 % When this new concept was combined with using e.g. DMAP catalyst, yield was still significantly increased, in this case to 96.3 % yield. Similarly, the process which applies the organic base catalyst has an enhanced assay.

### Example 8:

### Large Scale Industrial Process according to the Invention

An industrial reactor containing a 1220 kg of dry dichloromethane was charged with 182.7 kg 2,2,2-trichloroethyl chloroformate (TCECF) at 20 ± 5 °C. The mixture was heated to reflux and refluxed for at least 3 hours. After the mixture was cooled to 20 ± 5 °C. The reactor was charged with 1022 g of DMAP at 20 ± 5 °C. The obtained mixture was then stirred at 20 ± 5 °C for at least 1 hour. Then the reactor was charged with 51 kg of DHLSMe at 20 ± 5 °C dissolved in dry dichloromethane (720 kg). The obtained mixture was then heated to reflux and refluxed for 30 hours. After the reaction mixture was cooled to 10 ± 5 °C and filtered through a pad of alumina. Fractions containing a product were collected, concentrated and cooled 0 ± 5 °C. Then heptane was added to the concentrated solution and the precipitated product was filtered on filter-dryer. After drying 75.1 kg (94 %) of DHLSMe-F is obtained. DHLSMe-F assay is 100 % determined by HPLC.

| Kinetics of the reaction: | | |
|---|---|---|
| t[h] | DHLSMe [HPLC A%] | DHLSMe-F [HPLC A%] |
| 0 | 100 | 0 |
| 4 | 35,7 | 64,30 |
| 9 | 83,05 | 16,95 |
| 26 | 5,86 | 94,14 |
| 30 | 5.89 | 94,11 |

## Claims

1. A process for producing *N*-6 demethylated 9,10-dihydrolysergic acid alkyl ester, comprising a step of *N*-6 demethylation of a 9,10-dihydrolysergic acid alkyl ester with a chloroformate, wherein the *N*-6 demethylation step is performed in the presence of an organic base as an organic catalyst.

2. The process according to claim 1, **characterized in that** the chloroformate is defined by the formula 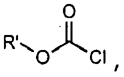 wherein R' is a substituted or unsubstituted hydrocarbon group, particularly wherein R' is defined by formula IV or V 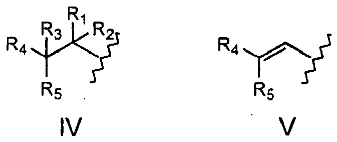 wherein R₁ and R₂, are the same or different and can be hydrogen atoms or are selected from halogens atoms (F, Cl, Br, I),
and wherein R₃, R₄, and R₅, are the same or different and can be hydrogen atoms or are selected from halogens atoms (F, Cl, Br, I), optionally substituted C₁ - C₈ alkyl, optionally substituted C₃-C₆ cycloalkyl, optionally substituted C₁ - C₈ alkenyl, and optionally substituted C₅-C₆ cycloalkenyl or aryl.

3. The process according to any one of the preceding claims, **characterized in that** the organic catalyst is an organic amine, preferably selected from the group consisting of sterically hindered organic amines, secondary and tertiary organic amines, and heterocyclic organic amines.

4. The process according to any one of the preceding claims, **characterized in that** the organic catalyst is selected from the group of compounds consisting of dimethylaminopyridine (DMAP) and DMAP analogues of the following general formulae: 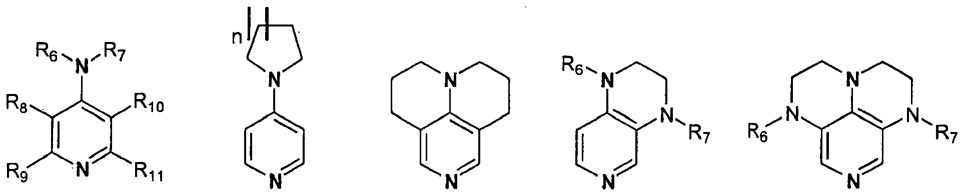 wherein R6 to R11 independently from each other denote H or C1 to C6 alkyl group, and n is 1, 2, 3, 4, 5 or 6,
wherein preferably the organic catalyst is 4-(dimethylamino)pyridine (DMAP).

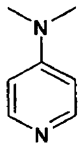

5. A process for converting a compound of formula I (DHLSAlkyl) into a compound of formula II (DHLSAlkyl-F), 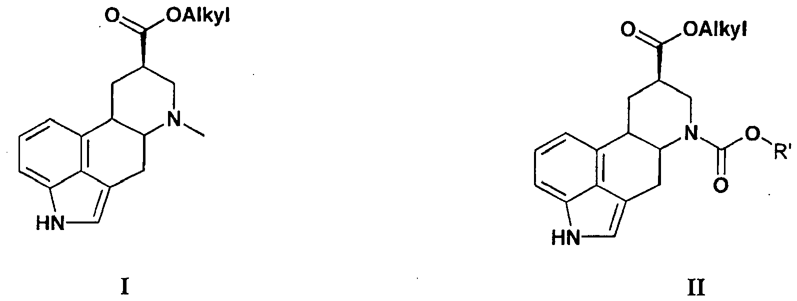 wherein R' is a substituted or unsubstituted hydrocarbon group, preferably a group as defined in claim 2 by formula IV or V,
the process comprising the steps of:
▪ providing a reaction medium free of water and/or HCl, and
▪ reacting DHLSAlkyl with chloroformate being defined by the formula 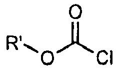 in said reaction medium free of water and/or HCl, optionally in the presence of an organic base as an organic catalyst, to obtain the compound of formula II (DHLSAlkyl-F).
wherein at least one additional water and/or HCl removal step is performed.

6. The process according to claim 5, wherein
a) before DHLSAlkyl is added, said chloroformate is added to the reaction medium, and the reaction medium is refluxed,
b) the reaction medium is optionally purged with nitrogen or subjected to evacuation,
c) DHLSAlkyl is added to the above mixture resulting from a) and optionally b)
d) reacting the obtained mixture for a time sufficient to give *N*-6 demethylated product.

7. The process according to claim5 or 6, further comprising the step of cleaving of the carbamate group of the compound of formula II (DHLSAlkyl-F) to give the compound of formula III (H-DHLSAlkyl):

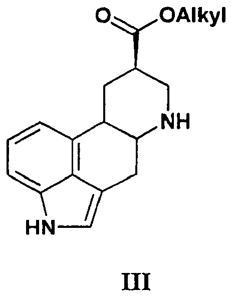

8. The process according to claim 1, characterized that the ratio of chloroformate used in total compared to 9,10-dihydrolysergic acid alkyl ester used in total for the step of *N*-6 demethylation is less than 5 equivalents to 1 equivalent, respectively.

9. The process according to any one of the preceding claims, which is performed at a large scale by using an amount of 9,10-dihydrolysergic acid alkyl ester (compound of formula I; DHLSAlkyl) above 1 kg, preferably wherein the demethylation step to obtain the compound of formula II (DHLSAlkyl-F) is terminated in less than 30 hours, preferably in less then 24 hours.

10. The process according to any one of the preceding claims, wherein chloroformate is continuously added to the reaction system.

11. A process for the preparation of ergot alkaloid drugs, comprising a process as defined in any one of claims 1 to 10.

12. A process for preparing an ergot alkaloid drug comprising carrying out a process as defined in claim 1 or claim 5 for obtaining an intermediate compound of formula II (DHLSAlkyl-F) or an intermediate compound of formula III (H-DHLSAlkyl), 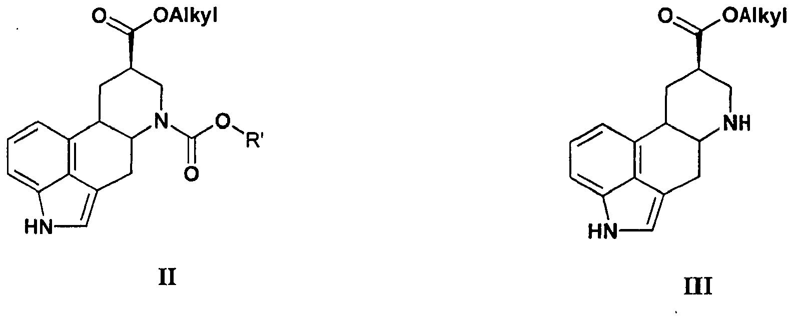 said intermediate compound of formula II (DHLSAlkyl-F) or of formula III (H-DHLSAlkyl) being free of any one of impurities of formula 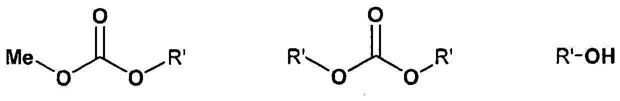 wherein R' is a substituted or unsubstituted hydrocarbon group, particularly wherein R' is defined by formula IV or V 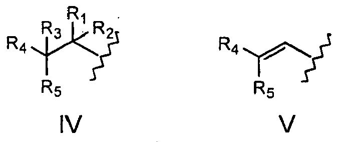 wherein R₁ and R₂, are the same or different and can be hydrogen atoms or are selected from halogens atoms (F, Cl, Br, I),
and wherein R₃, R₄, and R₅, are the same or different and can be hydrogen atoms or are selected from halogens atoms (F, Cl, Br, I), optionally substituted C₁ - C₈ alkyl, optionally substituted C₃-C₆ cycloalkyl, optionally substituted C₁ - C₈ alkenyl, and optionally substituted C₅-C₆ cycloalkenyl or aryl;
and carrying our further synthesis steps to obtain the ergot alkaloid drug.

13. The process according to claim 11 or 12, wherein the ergot alkaloid drug is cabegoline or pergolide.

## Patentansprüche

1. Verfahren zum Herstellen von *N*-6 demethyliertem 9,10-Dihydrolysergsäurealkylester, das einen Schritt der *N*-6 Demethylierung eines 9,10-Dihydrolysergsäurealkylesters mit einem Chlorformiat umfasst, wobei der *N*-6-Demethylierschritt in Gegenwart einer organischen Base als einem organischen Katalysator ausgeführt wird.

2. Verfahren gemäß Anspruch 1, **dadurch gekennzeichnet, dass** das Chlorformiat durch die Formel 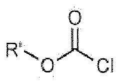 definiert ist, wobei R' eine substituierte oder unsubstituierte Kohlenwasserstoffgruppe ist, insbesondere wobei R' durch die Formel IV oder V definiert ist 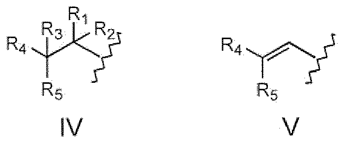 worin R₁ und R₂ gleich oder verschieden sind und Wasserstoffatome sein können oder aus Halogenatomen (F, Cl, Br, I) ausgewählt sind,
und worin R₃, R₄ und R₅ gleich oder verschieden sind und Wasserstoffatome sein können oder aus Halogenatomen (F, Cl, Br, I), wahlweise substituiertem C₁-C₈-Alkyl, wahlweise subsubstituiertem C₃-C₆-Cycloalykl, wahlweise substituiertem C₁-C₈-Alkenyl und wahlweise substituiertem C₅-C₆-Cycloalkenyl oder Aryl ausgewählt sind.

3. Verfahren gemäß irgendeinem der vorangehenden Ansprüche, **dadurch gekennzeichnet, dass** der organische Katalysator ein organisches Amin ist, vorzugsweise aus der aus steirisch gehinderten organischen Aminen, sekundären und tertiären organischen Aminen sowie heterocyclischen organischen Aminen bestehenden Gruppe ausgewählt ist.

4. Verfahren gemäß irgendeinem der vorangehenden Ansprüche, **dadurch gekennzeichnet, dass** der organische Katalysator aus der Gruppe von Verbindungen ausgewählt ist, die aus Dimethylaminopyridin (DMAP) und DMAP-Analoga der folgenden allgemeinen Formel besteht: 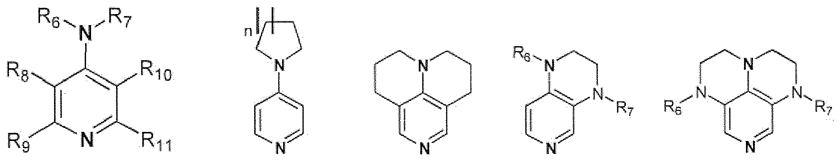 worin R6 bis R11 unabhängig voneinander H oder C1 bis C6-Alkylgruppe bezeichnen und n gleich 1, 2, 3, 4, 5 oder 6 ist,
wobei vorzugsweise der organische Katalysator 4-(Dimethylamino)pyridin (DMAP) ist:

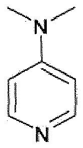

5. Verfahren zum Umwandeln einer Verbindung der Formel I (DHLSAlkyl) in eine Verbindung der Formel II (DHLSAlkyl-F), 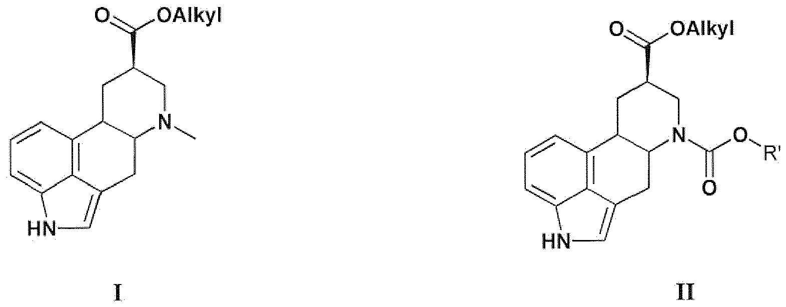 wobei R' eine substituierte oder unsubstituierte Kohlenwasserstoffgruppe ist, vorzugsweise eine wie in Anspruch 2 durch Formel IV oder V definierte Gruppe,
wobei das Verfahren die Schritte umfasst:
• Bereitstellen eines Reaktionsmediums, das frei von Wasser und/oder HCl ist, und
• Umsetzen von DHLSAlkyl mit Chlorformiat, welches durch die Formel 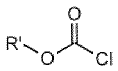 definiert ist, in besagtem Reaktionsmedium, welches frei von Wasser und/oder HCl ist, wahlweise in Gegenwart einer organischen Base als einem organischen Katalysator, um die Verbindung der Formel II (DHLSAlkyl-F) zu erhalten,
wobei mindestens ein zusätzlicher Wasser- und/oder HCl-Entfernungsschritt ausgeführt wird.

6. Verfahren gemäß Anspruch 5, bei dem
a) bevor DHLSAlkyl zugegeben wird, das besagte Chlorformiat zum Reaktionsmedium zugegeben wird und das Reaktionsmedium unter Rückfluss gesetzt wird,
b) das Reaktionsmedium wahlweise mit Stickstoff durchblasen wird oder einer Evakuierung unterzogen wird,
c) DHLSAlkyl zu der obigen, aus a) und wahlweise b) resultierenden Mischung zugegeben wird,
d) Umsetzen der erhaltenen Mischung für eine Dauer, die zum Erhalt von *N*-6 demethyliertem Produkt ausreichend ist.

7. Verfahren gemäß Anspruch 5 oder 6, ferner umfassend den Schritt des Spaltens der Carbamatgruppe der Verbindung der Formel II (DHLSAlkyl-F), um die Verbindung der Formel III (H-DHLSAlkyl) zu ergeben:

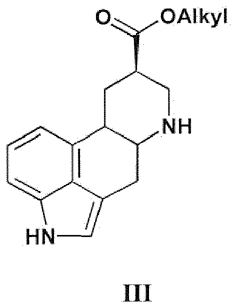

8. Verfahren gemäß Anspruch 1, **dadurch gekennzeichnet, dass** das Verhältnis von insgesamt verwendetem Chlorformiat im Vergleich zum insgesamt verwendten 9,10-Dihydrolysergsäurealkylester des Schritts der N-6-Demethylierung geringer als jeweils 5 Äquivalente zu einem Äquivalent ist.

9. Verfahren gemäß irgendeinem der vorangehenden Ansprüche, welches in großem Maßstab durch Verwendung einer Menge von 9,10-Dihydrolysergsäurealkylester (Verbindung der Formel I; DHLSAlkyl) von über 1 kg ausgeführt wird, vorzugsweise wobei der Demethylierschritt zum Erhalt der Verbindung der Formel II (DHLSAlkyl-F) in weniger als 30 Stunden, vorzugsweise in weniger als 24 Stunden beendet wird.

10. Verfahren gemäß irgendeinem der vorangehenden Ansprüche, wobei Chlorformiat kontinuierlich zum Reaktionssystem zugegeben wird.

11. Verfahren zur Herstellung von Ergot-Alkaloid-Wirkstoffen, umfassend ein Verfahren wie in irgendeinem der Ansprüche 1 bis 10 definiert.

12. Verfahren zum Herstellen eines Ergot-Alkaloid-Wirkstoffs umfassend das Ausführen eines Verfahrens wie in Anspruch 1 oder Anspruch 5 definiert zum Erhalt einer Zwischenverbindung der Formel II (DHLSAlkyl-F) oder einer Zwischenverbindung der Formel III (H-DHLSAlkyl), 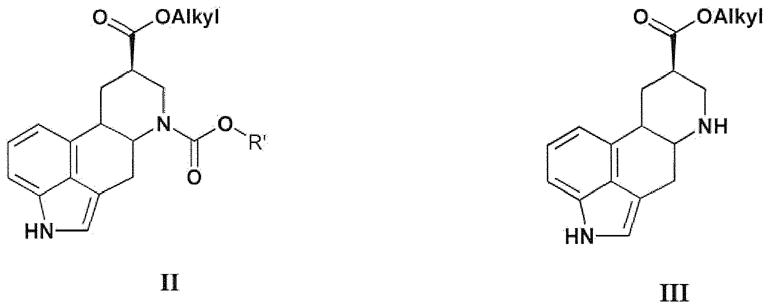 wobei die besagte Zwischenverbindung der Formel II (DHLSAlkyl-F) oder Formel III (H-DHLSAlkyl) frei ist von irgendeiner der Verunreinigungen der Formel 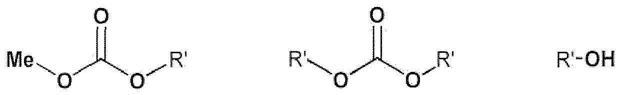 worin R' eine substituierte oder unsubstituierte Kohlenwasserstoffgruppe ist, insbesondere worin R' durch Formel IV oder V definiert ist 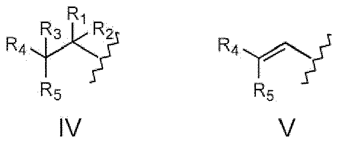 worin R₁ und R₂ gleich oder verschieden sind und Wasserstoffatome sein können oder aus Halogenatomen (F, Cl, Br, I) ausgewählt sind,
und worin R₃, R₄ und R₅ gleich oder verschieden sind und Wasserstoffatome sein können oder aus Halogenatomen (F, Cl, Br, I), wahlweise substuiertem C₁-C₈-Alkyl, wahlweise substituiertem C₃-C₆-Cycloalkyl, wahlweise substituiertem C₁-C₈-Alkenyl und wahlweise substituiertem C₅-C₆-Cycloalkenyl oder Aryl ausgewählt sind;
und Ausführen von weiteren Syntheseschritten, um den Ergot-Alkaloid-Wirkstoff zu erhalten.

13. Verfahren gemäß Anspruch 11 oder 12, wobei der Ergot-Alkaloid-Wirkstoff Cabegolin oder Pergolid ist.

## Revendications

1. Procédé de production d'ester alkylique d'acide 9,10-dihydrolysergique déméthylé en *N*-6, comprenant une étape de déméthylation en *N-6* d'un ester alkylique d'acide 9,10-dihydrolysergique avec un chloroformiate, dans lequel l'étape de déméthylation en *N-6* est réalisée en présence d'une base organique en tant que catalyseur organique.

2. Procédé selon la revendication 1, **caractérisé en ce que** le chloroformiate est défini par la formule 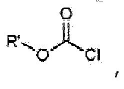 dans laquelle R' est un groupe hydrocarbure substitué ou non substitué, en particulier dans laquelle R' est défini par la formule IV ou V 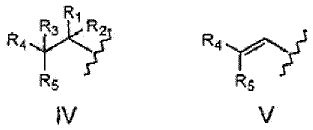 où R₁ et R₂ sont identiques ou différents et peuvent être des atomes d'hydrogène ou sont choisis parmi des atomes d'halogène (F, Cl, Br, I),
et où R₃, R₄ et R₅ sont identiques ou différents et peuvent être des atomes d'hydrogène ou sont choisis parmi des atomes d'halogène (F, Cl, Br, I), alkyle en C₁ à C₈ facultativement substitué, cycloalkyle en C₃ à C₆ facultativement substitué, alcényle en C₁ à C₈ facultativement substitué et cycloalcényle en C₅ à C₆ facultativement substitué ou aryle.

3. Procédé selon l'une quelconque des revendications précédentes, **caractérisé en ce que** le catalyseur organique est une amine organique, de préférence choisie dans le groupe consistant en les amines organiques stériquement encombrées, les amines organiques secondaires et tertiaires, et les amines organiques hétérocycliques.

4. Procédé selon l'une quelconque des revendications précédentes, **caractérisé en ce que** le catalyseur organique est choisi dans le groupe des composés consistant en la diméthylaminopyridine (DMAP) et des analogues de DMAP de formules générales suivantes : 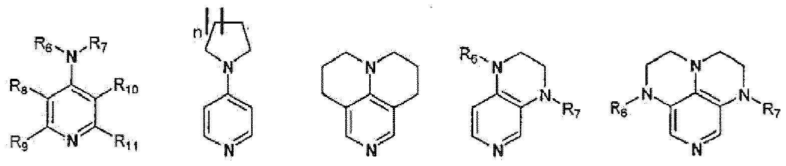 dans lesquelles R₆ à R₁₁ indépendamment les uns des autres désignent H ou un groupe alkyle en C₁ à C₆, et n vaut 1, 2, 3, 4, 5 ou 6,
dans lequel de préférence le catalyseur organique est la 4-(diméthylamino)pyridine (DMAP),

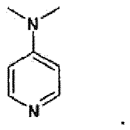

5. Procédé de conversion d'un composé de formule I (DHLSAlkyle) en un composé de formule II (DHLSAlkyle-F), 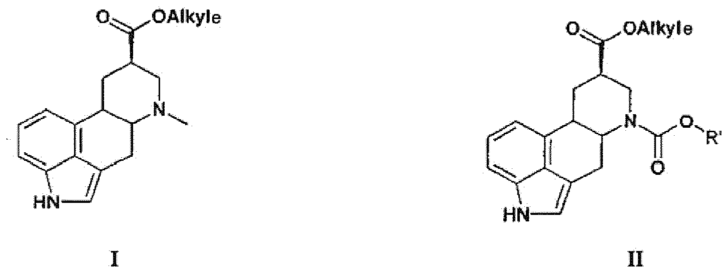 dans lesquelles R' est un groupe hydrocarbure substitué ou non substitué, de préférence un groupe tel que défini dans la revendication 2 par la formule IV ou V,
le procédé comprenant les étapes de :
- fourniture d'un milieu de réaction dépourvu d'eau et/ou de HCl, et
- mise en réaction de DHLSAlkyle avec du
chloroformiate qui est défini par la formule 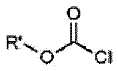 dans ledit milieu de réaction dépourvu d'eau et/ou de HCl, facultativement en présence d'une base organique en tant que catalyseur organique, pour obtenir le composé de formule II (DHLSAlkyle-F),
dans lequel au moins une étape additionnelle d'élimination d'eau et/ou de HCl est réalisée.

6. Procédé selon la revendication 5, dans lequel
a) avant que le DHLSAlkyle soit ajouté, ledit chloroformiate est ajouté au milieu de réaction, et le milieu de réaction est mis au reflux,
b) le milieu de réaction est facultativement purgé avec de l'azote ou soumis à une évacuation,
c) du DHLSAlkyle est ajouté au mélange ci-dessus résultant de a) et facultativement de b)
d) mise en réaction du mélange obtenu pendant un temps suffisant pour donner un produit déméthylé en *N-*6.

7. Procédé selon la revendication 5 ou 6, comprenant en outre l'étape de clivage du groupe carbamate du composé de formule II (DHLSAlkyle-F) pour donner le composé de formule III (H-DHLSAlkyle) :

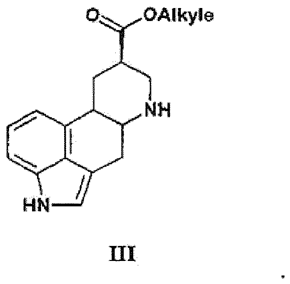

8. Procédé selon la revendication 1, **caractérisé en ce que** le rapport de chloroformiate utilisé au total comparé à l'ester alkylique d'acide 9,10-dihydrolysergique utilisé au total pour l'étape de déméthylation en *N-6* est de moins de 5 équivalents pour 1 équivalent, respectivement.

9. Procédé selon l'une quelconque des revendications précédentes, qui est réalisé à grande échelle en utilisant une quantité d'ester alkylique d'acide 9,10-dihydrolysergique (composé de formule I ; DHLSAlkyle) au-delà de 1 kg, de préférence dans lequel l'étape de déméthylation pour obtenir le composé de formule II (DHLSAlkyle-F) est terminée en moins de 30 heures, de préférence en moins de 24 heures.

10. Procédé selon l'une quelconque des revendications précédentes, dans lequel du chloroformiate est ajouté continuellement au système de réaction.

11. Procédé de préparation de médicaments alcaloïdes de l'ergot de seigle, comprenant un procédé tel que défini dans l'une quelconque des revendications 1 à 10.

12. Procédé de préparation d'un médicament alcaloïde de l'ergot de seigle, comprenant la réalisation d'un procédé tel que défini dans la revendication 1 ou la revendication 5 pour obtenir un composé intermédiaire de formule II (DHLSAlkyle-F) ou un composé intermédiaire de formule III (H-DHLSAlkyle), 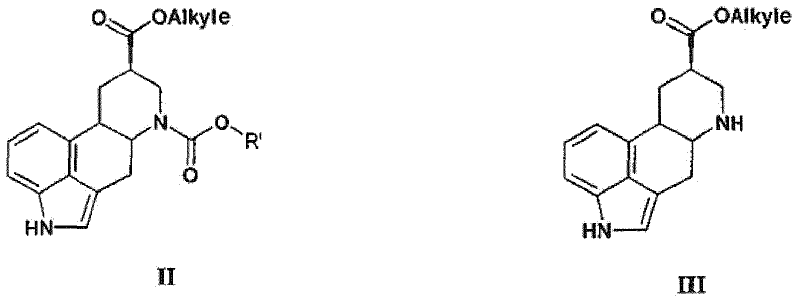 ledit composé intermédiaire de formule II (DHLSAlkyle-F) ou de formule III (H-DHLSAlkyle) étant dépourvu de l'une quelconque des impuretés de formule 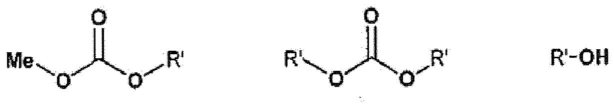 où R' est un groupe hydrocarbure substitué ou non substitué, en particulier où R' est défini par la formule IV ou V 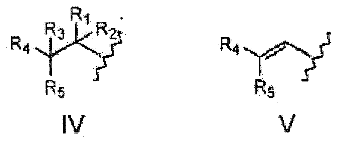 où R₁ et R₂ sont identiques ou différents et peuvent être des atomes d'hydrogène ou sont choisis parmi des atomes d'halogène (F, Cl, Br, I),
et où R₃, R₄ et R₅ sont identiques ou différents et peuvent être des atomes d'hydrogène ou sont choisis parmi des atomes d'halogène (F, Cl, Br, I), alkyle en C₁ à C₈ facultativement substitué, cycloalkyle en C₃ à C₆ facultativement substitué, alcényle en C₁ à C₈ facultativement substitué et cycloalcényle en C₅ à C₆ facultativement substitué ou aryle ;
et la réalisation des étapes de synthèse supplémentaires pour obtenir le médicament alcaloïde de l'ergot de seigle.

13. Procédé selon la revendication 11 ou 12, dans lequel le médicament alcaloïde de l'ergot de seigle est la cabergoline ou le pergolide.
